# EUROPEAN PATENT SPECIFICATION

(11) **EP 0 517 435 B1**
(45) Date of publication and mention of the grant of the patent: **05.11.1997**
(21) Application number: 92304882.1
(22) Date of filing: 29.05.1992
(51) Int. Cl.: A61B 17/72

(54) **Intramedullary osteosynthetic device**
Intramedulläre Vorrichtung zur Osteosynthese
Dispositif intramédullaire pour l'ostéosynthèse

(30) Priority: 01.06.1991 GB 9111826
(43) Date of publication of application: 09.12.1992
(73) Proprietor: HALIFAX ORTHOPAEDIC RESEARCH LIMITED, Halifax, West Yorkshire HX1 2BX (GB)
(72) Inventor: Halder, Subhash Chandra, Halifax, West Yorkshire (GB)
(74) Representative: Neill, Alastair William

(56) References cited:
- EP-A- 0 094 489
- EP-A- 0 226 701
- DE-A- 2 519 488
- FR-A- 2 484 243
- FR-A- 2 606 269
- FR-A- 2 622 788
- US-A- 2 327 434
- US-A- 2 998 007
- UNFALLCHIRURGIE,Band II 1980 Verlag Dr. med. D. Straube, Erlangen

## Description

The present invention relates to apparatus for the support of a fractured or otherwise damaged bone.

In recent years there has been an increasing tendency to treat fractures of bones by supporting the bone itself either externally by means of a plate or internally by means of a nail rather than by supporting the arm as a whole with a splint or plaster cast.

For example, known apparatus for the support of a fractured humerus typically comprises an elongate, hollow stainless steel rod, commonly referred to as a "nail". In use, the nail is inserted into the medullary canal of the humerus through an opening made in the head of the humerus, ie. at the shoulder. In order to prevent rotational movement of parts of the humerus at either side of the fracture relative to one another the nail is locked in position distally (ie. at the elbow end) and proximally (ie. at the shoulder end).

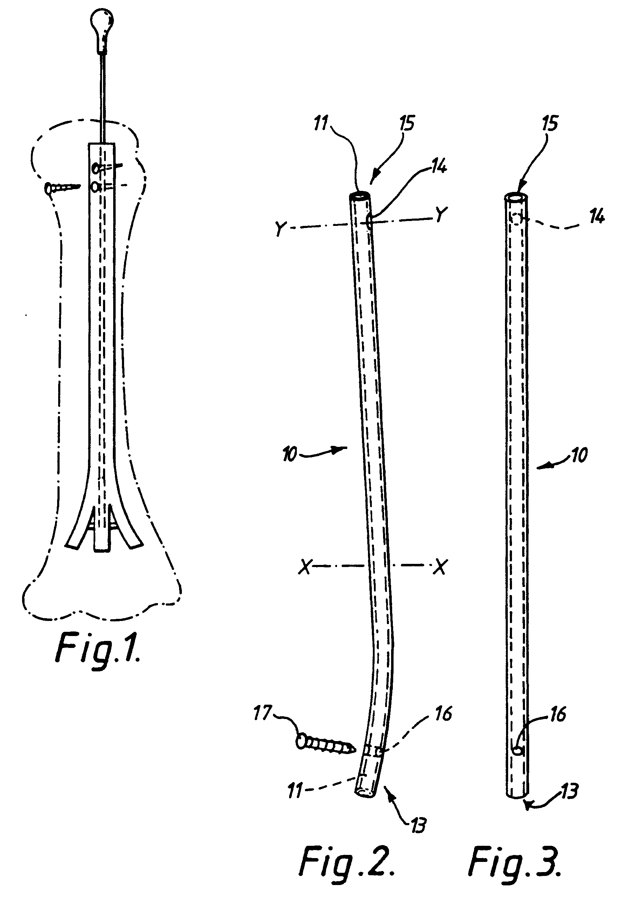

Distal locking is achieved by an expandable head at the distal end of the nail. A long screwdriver is inserted down the hollow nail to turn a spreading screw and expand the head (see Figure 1). Proximal locking is achieved by inserting screws through the upper arm transverse to the longitudinal axis of the humerus and nail, and passing through both the bone and the nail.

A disadvantage with the known system is that the patient suffers considerable discomfort due to the muscles in the patients shoulder and upper arm remaining stiff and sore for some time after the operation. The reason for this stiffness and soreness is that the initial incision before insertion of the nail must be made in the head of the humerus, which involves disturbing and/or making a hole in the deltoid muscles and or the short rotator muscle since there must be access for the screwdriver at the proximal end to expand the head of the nail at the distal end. The known operation therefore necessarily involves some damage to the upper arm and shoulder which results in soreness and a tendency for the patient not to move the shoulder due to this soreness. The inactivity of the muscles then results in stiffness.

A further disadvantage of the known device is that it may not be possible to remove the nail at a later stage, for example if the fracture heals totally. Bone or other material tends to form around the expanded head of the nail, so that even if a screwdriver is used to turn the spreading screw in the reverse direction, the head of the nail may remain expanded.

Furthermore, the anti-rotational grip provided by the expanded head is not particularly good.

Another known prior device for supporting a fractured bone is described in Unfallchirurgie, Band II 1980, Verlag Dr. Med. D.Straube, Erlangen. This discloses an elongate bone support for insertion in a bone, wherein elongate wires may be inserted at one end of the support, to project from the other end thereof into the bone, thereby to inhibit rotation of the bone.

A further prior device for supporting a fractured bone is disclosed in United States Patent No 2 998 007 (Herzog) in which a hollow support is provided for insertion into a bone, there being further provided one or more wires which are arranged to pass along the support until they reach the required distance in the bone, whereupon the wires are rotated to project through apertures in the support, and into the interior of the bone.

The present invention aims to provide apparatus for the support of a fractured bone which apparatus can be inserted in the fractured bone with less resultant discomfort to the patient, and which reduce or avoid other disadvantages of the prior art.

According to the invention, there is provided a bone support comprising a hollow elongate rod insertable along at least a substantial part of the length of the interior of a bone, and an elongate member extending within the rod, one end of the elongate member comprising a projection which can be extended to engage with the bone at one end of the rod thus acting as a bone rotation prevention means, and can thereafter be positively retracted, the elongate member being operable from the other end of the rod, the rod having a side aperture at a proximal end thereof through which the projection is arranged to pass in use, characterised in that the elongate member is arranged to cooperate with the rod such that if the elongate member is caused to slide along the rod, bevelled edges of the apertures of the rod cause the elongate member to be guided through the aperture in the wall of the rod such that the said one end of the elongate member is caused to extend away from the rod at an angle to the longitudinal extent of the rod.

There may be provided more than one projection and preferably, there are provided two projections arranged on substantially opposed sides of the bone support.

The projection may comprise an elongate, thin piercing member such as a wire.

Preferably, there are provided two substantially opposed side apertures in the rod.

In a preferred arrangement there is provided further bone rotation prevention means which comprises a screw arranged to pass through the bone and through the rod, substantially transverse to the longitudinal axis of the rod, to inhibit rotational movement of the bone.

Preferably, the first said bone rotation prevention means is arranged to inhibit rotational movement of a proximal end of the bone.

Preferably, the second said bone rotation prevention means is arranged to inhibit rotational movement of a distal end of the bone.

Preferably, the rod is generally clover-leaf shape in cross-section.

Preferably the other end of the elongate member is capable of manipulation from the side of a limb.

For example the said other end of the elongate member may be flexible so that it can project out of the rod in the longitudinal direction and then flex outwardly to the side of the limb.

Where two projections are used, they may be formed by two wires extending inside the hollow rod. The wires may be secured together, for example by brazing or welding, towards the distal end of the device. Alternatively the two projections may comprise portions of the same length of wire. The length of wire may be bent into two portions which may form a loop therebetween.

The rod may have at least one and preferably two, open ends.

Preferably, the distal end of the rod is open and the proximal end of the rod has the side aperture out of which the projection passes.

The two projections may be arranged to emerge from the two respective apertures at an acute angle between the projections.

At least one aperture may be shaped so as to guide the or each projection. In a preferred arrangement at least one aperture has at least one bevelled edge to guide the projection Preferably at least one aperture has a bevelled inside lower edge and a bevelled outside upper edge to guide the projection.

Preferably there is provided locating means comprising a substantially rigid elongate member arranged to be inserted in a bone prior to insertion of an elongate rod, the elongate rod being arranged, in use to extend over the locating means and be guided by cooperation with the locating means during insertion of the rod into the bone, the locating means being capable of being withdrawn from cooperation with the elongate rod subsequent to insertion of the elongate rod in the bone.

The locating means may comprise a portion of wire.

Preferably the locating means and the bone rotation prevention means comprise the same portion of wire.

In a preferred arrangement the elongate rod has a smoothly bent portion comprising substantially one third of its length. The smoothly bent portion is preferably the end of the rod which is located at the distal end of a humerus bone in use.

The distal third of the rod may be at an angle of 2° to 4° to the longitudinal axis of the remainder of the rod.

The rod may be twisted along its length. In a preferred arrangement an upper end of the rod is twisted through approximately 30° with respect to a lower end of the rod.

Specific embodiments will now be described, with reference to the accompanying drawings, in which:-
Figure 1 shows a known support for a fractured bone;
Figure 2 is a side view of a nail according to a first embodiment of the present invention;
Figure 3 shows the nail of Figure 2 viewed in the direction of arrow A in Figure 2;
Figure 4 is a cross section of the nail of Figure 2 taken along the line X-X of Figure 2;
Figure 5 is a cross section of the nail of Figure 2 taken along line Y-Y of Figure 2;
Figure 6 is a locking member for use with the nail of Figures 2 to 5;
Figure 7 shows the locking member of Figure 6 in the configuration it adopts inside the nail of Figures 2 to 5;
Figure 8 is a view of a nail and locking member partly inserted in a humerus;
Figure 9 is a view of a nail and locking member fully inserted in a humerus;
Figure 10 is a side view of an alternative embodiment of nail according to the invention;
Figure 11 is a cross section of the nail of Figure 10, shown without the locking member, taken along line Z-Z of Figure 10;
Figure 12 is a view of the nail and locking member of Figure 10, shown inserted in a fractured humerus;
Figure 13 shows a preferred embodiment of fixing wire;
Figure 14 shows, in detail, a cross-section through a portion of a wall of the nail; and
Figure 15 shows a further alternative embodiment of nail.

Referring particularly to Figures 2 and 3, a hollow, elongate open ended rod, referred to hereinafter as a nail, is shown generally at 10. Within the nail 10 there is a passage 11. Passage 11 extends the full length of the nail 10, there being an aperture 14 located on the surface of the nail 10 approximately 26 mm (1 inch) from the proximal end 15 of the nail 10. One third of the nail is smoothly bent at distal end 13, at an angle of 2° to 4° to the longitudinal axis of the rest of the nail.

At approximately 13mm (0.5 inch) from the distal end 13 of the nail 10 there is located a hole 16 passing through the nail 10 in a direction substantially transverse to the longitudinal axis of the nail 10, which hole 16, in use, receives a distal fixing screw 17 as described in more detail below.

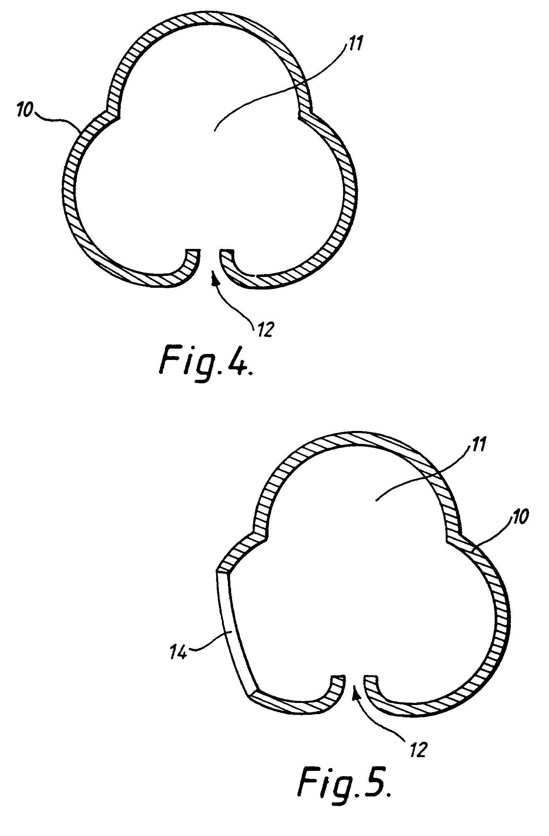

Figure 4 is a cross sectional view of the nail 10 of Figure 2 taken along the line X-X. The nail 10 is of a generally clover-leaf section with a slit 12 extending the full length of the nail. The purpose of the slit is to allow for some degree of compression of the nail, where necessary, inside the bone in use.

Figure 5 is the cross sectional view of the nail 10 of Figure 2 taken along the line Y-Y showing the aperture 14.

In use, a small aperture is formed in the supraolecranon fossa in the region of the elbow at the distal end of the humerus.

A round steel guide wire (not shown) is inserted into the aperture and is gently urged up the medullary canal to the desired distance, leaving one end of the guide wire protruding from the aperture.

The proximal end 15 of the nail 10 is then placed over the protruding end of the guide wire such that the guide wire enters the passage 11 of the nail 10. The nail 10 is then inserted in the medullary canal, its direction being guided by its cooperation with the guide wire. Once the nail is fully inserted in the humerus the guide wire is drawn out of the humerus through the open distal end 13 of the nail 10.

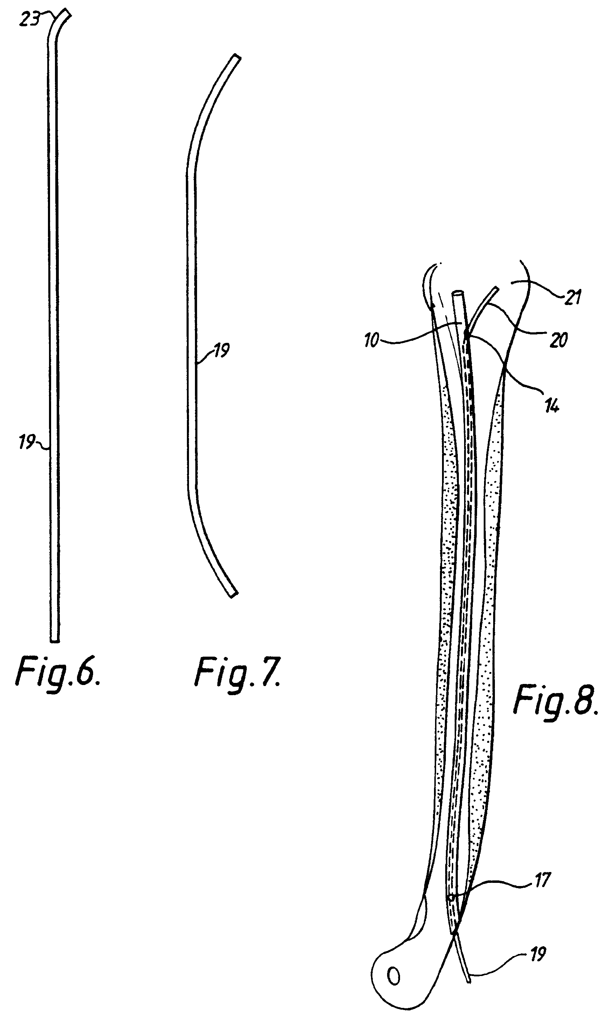

A round steel fixing wire 19 (as shown in Figure 6) is then inserted in the passage 11 of the nail 10. Figure 7 shows the fixing wire 19 in the bent configuration it adopts when fully inserted in the nail 10.

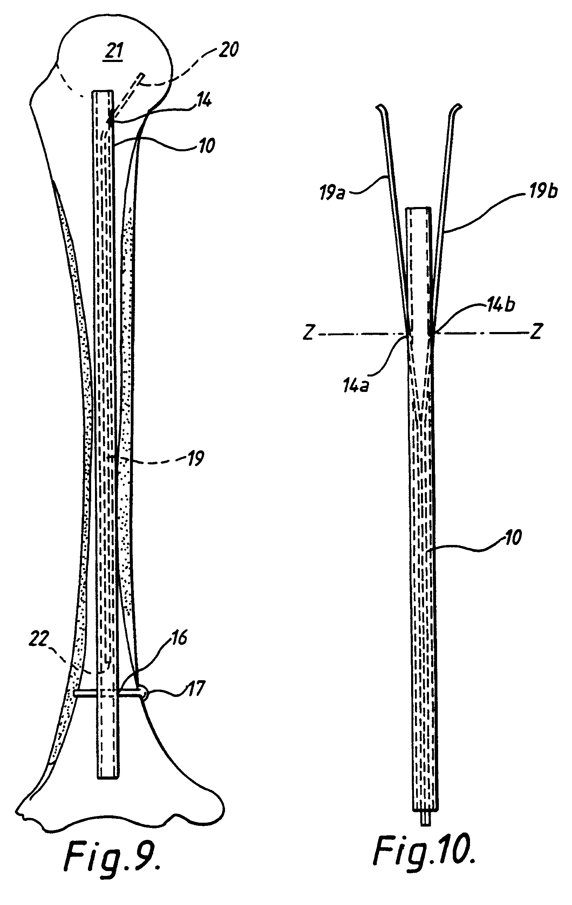

Figure 8 shows the fixing wire 19 being inserted in the nail 10. Figure 9 shows the fixing wire 19 fully inserted in the nail 10, the end 20 of the fixing wire protruding from the aperture 14 of the nail 10 and being lodged in the head of the humerus at region 21. When the wire 19 is fully inserted in the nail 10 a punch (not shown) is used to drive end 22 of wire 19 further into the nail 10 so that end 22 becomes located higher than hole 16.

The outward movement of the end 20 of the fixing wire has a piercing effect which causes minimal disturbance to the proximal end of the humerus but also very effectively locks the proximal end of the humerus to the nail, preventing rotation with respect to the nail.

Distal locking screw 17 is inserted through hole 16 such that it passes through the posterior cortex of the humerus to the anterior cortex of the humerus to inhibit rotational movement of the distal end of the humerus relative to the nail.

Once both the proximal end of the humerus and the distal end of the humerus are locked to the nail, they are obviously secured against rotation with respect to one another, thus giving the fracture the best chance of healing, regardless of the position of the fracture along the length of the humerus.

The guide wire may be used as the fixing wire simply by removing it from passage 11 of the inserted nail 10 and re-inserting it through passage 11 so that it emerges from aperture 14. An end portion 23 of guide wire may be bent after withdrawal from the passage 11 and prior to re-insertion to more easily enable it to locate and pass out through aperture 14.

Figure 9 shows the nail 10 fully inserted in the humerus, distal locking screw passing through hole 16 in the nail 10 to lock the distal end of the humerus in position.

Because the end 20 of the fixing wire is elongate and relatively thin, and operates by piercing the bone, it can subsequently be retracted again, even after a significant period of time. Even if healed bone is tending to grip the end 20 of the wire, only a small amount of retractive force is required, pulling on the other end of the wire to withdraw it from the bone. The nail, no longer locked to the bone, can then be withdrawn, the screw 17 of course first being removed.

It will be seen that with this embodiment of the invention, all the operations can be performed from the region of the elbow, with minimal incisions and minimal disturbance of bone and tissue.

Figure 10 snows a preferred embodiment of the device, in which there are two fixing wires, 19a and 19b. One wire emerges from the nail through a first aperture 14a and the other emerges through a second aperture 14b. Having two piercing projections, one at each side of the proximal end of the nail, substantially increases the grip on the proximal fragment of the humerus, thus preventing rotation even more effectively than in the first embodiment.

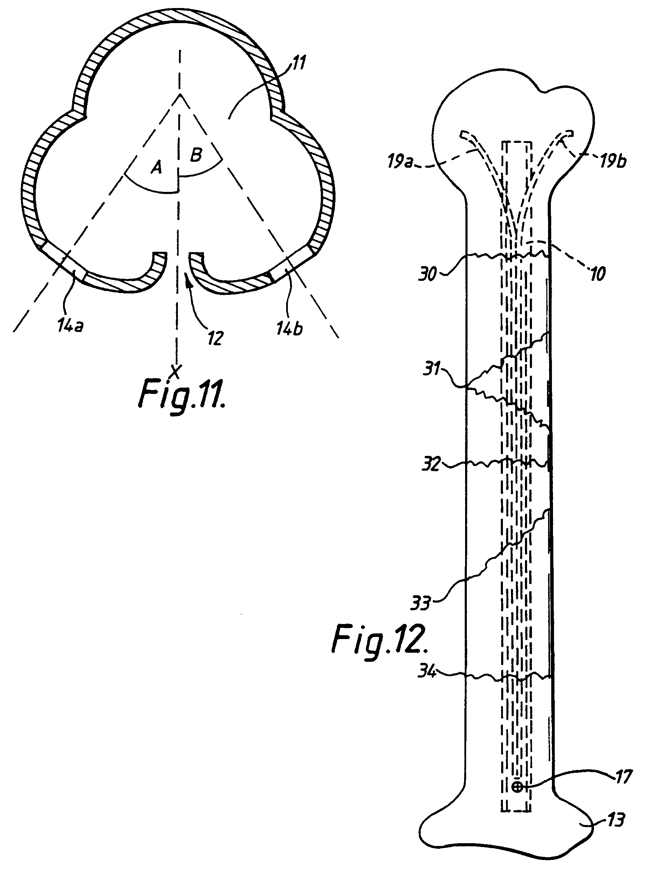

Figure 11 shows a cross-sectional view of the nail of the preferred embodiment of Figure 10, taken along line Z-Z of Figure 10, the fixing wires 19a, 19b having been removed. The axes of holes 14a and 14b make angles A and B respectively with axis X passing through the slit 12. Preferably angles A and B are both approximately 30°.

Figure 12 shows a humerus bone with the nail of Figure 10 inserted therein, showing the two fixing wires 19a, 19b embedded in the head of the bone. Fractures 30 to 34 are treatable by means of the nail, rotation of the bone relative to the nail being prevented by cooperation of the fixing wires 19a, 19b and of the locking screw 17 with the bone.

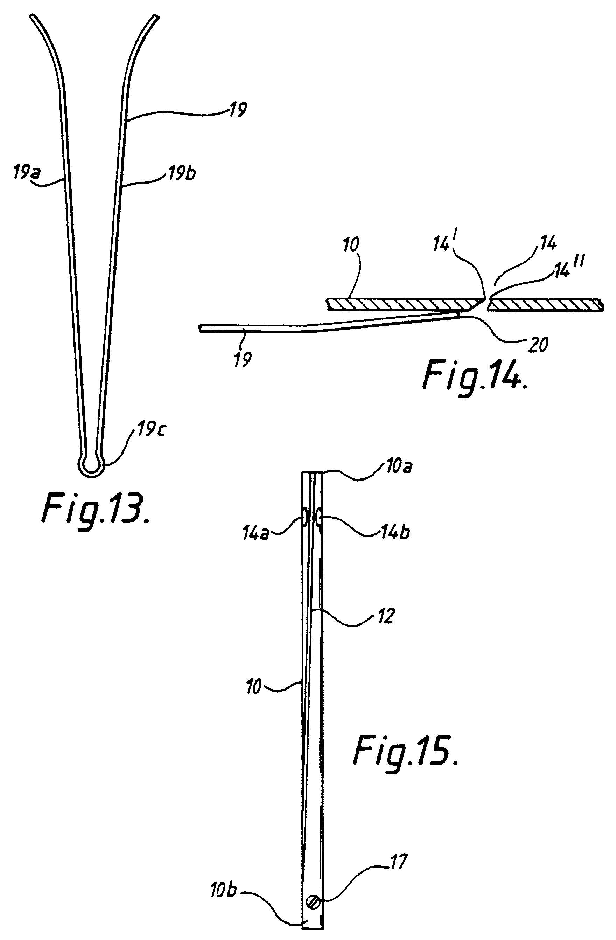

Towards the distal end of the nail, the wires 19a and 19b can be secured together, for example by brazing or welding. However, in a preferred embodiment shown in Figure 13 the wires 19a and 19b are actually separate limbs 19a and 19b of a common wire 19 which has been bent to form a loop 19c, through which the screw 17 (shown in Figure 12) is arranged to pass in use to secure the position of the wires 19a and 19b at the distal end. This arrangement conveniently obviates the need to drill a hole through a brazed joint of wires 19a and 19b in order that the screw 17 can be made to secure the wires 19a, 19b.

Figure 14 shows in detail a cross-section through a part of the wall of the nail 10, showing one of the holes 14. On a lower edge 14' of the hole 14, the inside surface is bevelled. On an upper edge 14'' of the hole 14 the outside surface is bevelled. The bevel on the upper edge 14'' is steeper than that on the lower edge 14'.

In use, as the wire 19 is pushed up through the nail 10 it first approaches the lower edge 14'. On contacting the bevelled surface of the lower edge 14' the tip 20 of the wire 19 is allowed to move outwardly, maintaining contact with the bevelled edge. Once the tip 20 contacts the upper edge 14'' the outside bevel urges the tip 20 out of the nail 10 through the aperture 14 to enable the tip 20 to become embedded in the head of the bone. The arrangement of the bevelled surfaces thus assists in the guiding of the tip 20 of the wire 19 out of the nail 10 through the hole 14.

Figure 15 shows a preferred embodiment of nail 10 in which the upper end 10a, where the holes 14a and 14b are located, has been twisted through approximately 30° with respect to the lower end 10b through which the screw 17 is inserted. It has been found that this arrangement assists in the more accurate location of the fixing wires (not shown) in the head of the humerus. In a preferred arrangement left and right handed nails are provided (not shown) in which the twist is in different directions for use in left and right handed bones, such as the left humerus and the right humerus.

Whilst the apparatus has been described above as supporting a humerus bone, it will be appreciated that the apparatus is readily adaptable for use in supporting other bones, such as other human bones or animal bones.

## Claims

1. A bone support comprising a hollow elongate rod (10) insertable along at least a substantial part of the length of the interior of a bone, and an elongate member (19) extending within the rod (10), one end of the elongate member (19) comprising a projection (20) which can be extended to engage with the bone at one end (15) of the rod thus acting as a bone rotation prevention means, and can thereafter be positively retracted, the elongate member (19) being operable from the other end (13) of the rod (10), the rod (10) having a side aperture (14) at a proximal end thereof through which the projection (20) is arranged to pass in use, characterised in that the elongate member (19) is arranged to cooperate with the rod (10) such that if the elongate member is caused to slide along the rod, bevelled edges of the apertures of the rod cause the elongate member to be guided through the aperture (14) in the wall of the rod such that the said one end (20) of the elongate member is caused to extend away from the rod at an angle to the longitudinal extent of the rod.

2. A bone support according to claim 1, wherein there are provided two projections (2) arranged on substantially opposed sides of the bone support (10).

3. A bone support according to claim 2, wherein there are provided two substantially opposed side apertures (14) in the rod.

4. A bone support according to any of claims 1 to 3, wherein there is provided further bone rotation prevention means which comprises a screw (17) arranged to pass through the bone and through the rod, substantially transverse to the longitudinal axis of the rod, to inhibit rotational movement of the bone.

5. A bone support according to claim 1, wherein the first said bone rotation prevention means is arranged to inhibit rotational movement of a proximal end (15) of the bone.

6. A bone support according to claim 4, wherein the second said bone rotation prevention means is arranged to inhibit rotational movement of a distal end (13) of the bone.

7. A bone support according to any one of the preceding claims, wherein the, or each, side aperture (14) has at least one bevelled edge to guide the projection (20).

## Patentansprüche

1. Knochenstützvorrichtung mit einer langen Hülse (10), die in wenigstens einen wesentlichen Teil des Inneren eines Knochens einführbar ist, wie mit einem innerhalb der Hülse (10) angeordneten langen Element (19), das einen Vorsprung (20) aufweist, der in eine solche Lage verbracht werden kann, daß er an einem Ende (15) der Hülse den Knochen erfaßt und somit als Knochenverdrehsicherung wirkt und sodann positiv zurückgezogen werden kann, daß das lange Element (19) vom anderen Ende (13) der Hülse (10) her manipulierbar ist und die Hülse (10) an einem proximalen Ende seitliche Öffnung (14) aufweist, durch welche der Vorsprung (20) beim Gebrauch hindurchgeführt wird, dadurch gekennzeichnet, daß das lange Element (19) derart gestaltet ist, daß es mit der Hülse (10) zusammenarbeitet, derart, daß beim Gleiten des langen Elementes entlang der Hülse angefaste Kanten der Öffnungen der Hülse das lange Element durch die Öffnung (14) in der Wand der Hülse führen, derart, daß sich das eine Ende (20) des langen Elementes unter einem Winkel zur Längserstreckung der Hülse von dieser hinwegerstreckt.

2. Knochenstützvorrichtung nach Anspruch 1, wobei zwei Vorsprünge (2) auf im wesentlichen einander gegenüberliegenden Seiten der Knochenstützvorrichtung (10) angeordnet sind.

3. Knochenstützvorrichtung nach Anspruch 2, wobei zwei im wesentlichen einander gegenüberliegende Seitenöffnungen (14) in der Hülse vorgesehen sind.

4. Knochenstützvorrichtung nach einem der Ansprüche 1 bis 3, wobei eine weitere Knochen-Verdrehsicherung vorgesehen ist, umfassend eine Schraube (17) zum Hindurchführen durch den Knochen sowie durch die Hülse quer zu deren Längsachse zum Unterbinden einer Drehbewegung des Knochens.

5. Knochenstützvorrichtung nach Anspruch 1, wobei die erste Knochen-Verdrehsicherung derart gestaltet ist, daß sie eine Drehbewegung eines proximalen Endes (15) des Knochens unterbindet.

6. Knochenstützvorrichtung nach Anspruch 4, wobei die genannte zweite Knochen-Verdrehsicherung derart gestaltet ist, daß sie eine Drehbewegung eines distalen Endes (13) des Knochens unterbindet.

7. Knochenstützvorrichtung nach einem der vorausgegangenen Ansprüche, wobei die einzelne Seitenöffnung (14) wenigstens eine angefaste Kante zum Führen des Vorsprunges (20) aufweist.

## Revendications

1. Support pour os comprenant une tige allongée creuse (10) susceptible d'être insérée le long d'au moins une partie substantielle de la longueur de l'intérieur d'un os, et un élément allongé (19) s'étendant à l'intérieur de la tige (10), une extrémité de l'élément allongé (19) comprenant un élément dépassant (20) pouvant être étendu pour venir en prise avec l'os à une extrémité (15) de la tige, servant ainsi de moyen empêchant la rotation de l'os, et pouvant ensuite être positivement rétracté, l'élément allongé (19) pouvant être actionné à partir de l'autre extrémité (13) de la tige (10), la tige (10) présentant une ouverture latérale (14) à une extrémité proximale de celle-ci, à travers laquelle l'élément dépassant (20) est amené à passer en cours d'utilisation, caractérisé en ce que l'élément allongé (19) est conçu pour coopérer avec la tige (10) de telle sorte que, si l'élément allongé est amené à glisser le long de la tige, des bords en biseau des ouvertures de la tige amènent l'élément allongé à être guidé à travers l'ouverture (14) ménagée dans la paroi de la tige, de telle sorte que l'extrémité (20) de l'élément allongé est amenée à se détourner de la tige, en formant un angle par rapport à la dimension longitudinale de la tige.

2. Support pour os selon la revendication 1, dans lequel sont prévues deux saillies (2) sur des côtés sensiblement opposés du support pour os (10).

3. Support pour os selon la revendication 2, dans lequel sont prévues, dans la tige, deux ouvertures latérales (14) sensiblement opposées.

4. Support pour os selon l'une quelconque des revendications 1 à 3, dans lequel est prévu un autre moyen empêchant la rotation de l'os, qui comprend une vis (17) conçue pour traverser l'os et la tige, de façon sensiblement transversale à l'axe longitudinal de la tige, afin d'empêcher un mouvement de rotation de l'os.

5. Support pour os selon la revendication 1, dans lequel le premier moyen empêchant la rotation de l'os est conçu pour empêcher le mouvement de rotation d'une extrémité proximale (15) de l'os.

6. Support pour os selon la revendication 4, dans lequel le deuxième moyen empêchant la rotation de l'os est conçu pour empêcher le mouvement de rotation d'une extrémité distale (13) de l'os.

7. Support pour os selon l'une quelconque des revendications précédentes, dans lequel l'ouverture latérale (14), ou chaque ouverture latérale, présente au moins un bord en biseau pour guider l'élément dépassant (20).
